Europäisches Patentamt

European Patent Office

Office européen des brevets

(19)

(11) Veröffentlichungsnummer : **0 030 330**
**B1**

(12) **EUROPÄISCHE PATENTSCHRIFT**

(45) Veröffentlichungstag der Patentschrift :
24.08.83

(21) Anmeldenummer : 80107412.1

(22) Anmeldetag : 27.11.80

(51) Int. Cl.³ : **C 07 C 67/56**, C 07 C 69/44,
C 07 C 67/48

(54) **Verfahren zur Reinigung von Aldehyde und/oder Acetale enthaltenden Carbonsäureestern.**

(30) Priorität : 06.12.79 DE 2949073

(43) Veröffentlichungstag der Anmeldung :
17.06.81 Patentblatt 81/24

(45) Bekanntmachung des Hinweises auf die Patenterteilung : 24.08.83 Patentblatt 83/34

(84) Benannte Vertragsstaaten :
BE CH DE FR GB IT LI NL

(56) Entgegenhaltungen :
CHEMICAL ABSTRACTS, Band 89, Heft 17, 23.
Oktober 1978, Seite 567, Zusammenfassung
146441d, COLUMBUS, OHIO (US)

(73) Patentinhaber : BASF Aktiengesellschaft
Carl-Bosch-Strasse 38
D-6700 Ludwigshafen (DE)

(72) Erfinder : Kummer, Rudolf, Dr.
Kreuzstrasse 6
D-6710 Frankenthal (DE)
Erfinder : Taglieber, Volker, Dr.
Beuthener Strasse 7
D-6700 Ludwigshafen (DE)
Erfinder : Weiss, Franz-Josef, Dr.
Schilfweg 1
D-6701 Neuhofen (DE)
Erfinder : Schneider, Heinz-Walter, Dr.
Bruesseler Ring 43
D-6700 Ludwigshafen (DE)

Verfahren zur Reinigung von Aldehyde und/oder Acetale enthaltenden Carbonsäureestern

Carbonsäureester lassen sich technisch herstellen durch Carbonylierung von Olefinen, z. B. durch Umsetzen von Äthylen, Propylen oder Butylen mit Kohlenmonoxid und Alkanolen in Gegenwart von Carbonylkomplexen von Metallen der VIII Gruppe des Periodensystems. Verwendet man als Ausgangsstoffe Diolefine, z. B. 1.3-Butadien, so erhält man über die Zwischenstufe Pentensäureester Adipinsäureester, die ein wertvolles Ausgangsprodukt für Faserrohstoffe darstellen. Da Kohlenmonoxid häufig in geringen Mengen Wasserstoff enthält oder durch mit eingeschlepptem Wasser in der Reaktion durch Konvertierung sich Wasserstoff bildet, tritt neben der Carbonylierungsreaktion auch eine Hydroformylierungsreaktion ein. Diese führt zu Aldehyden und durch Umsetzung mit vorhandenen Alkoholen zu Acetalen. Sobald die Siedepunkte der Acetale und Aldehyde mit den erzeugten Estern sehr eng beieinander liegen, ist eine destillative Abtrennung der unerwünschten Aldehyde und Acetale technisch außerordentlich aufwendig. Die Abtrennung von Aldehyden und Acetalen hat besondere Bedeutung bei der Herstellung von Adipinsäureestern, da die daraus hergestellte Adipinsäure dann Monocarbonsäuren enthält und somit eine für die Herstellung von Polymeren wenig geeignete Qualität aufweist. Darüber hinaus führen selbst geringe Mengen an Aldehyden und Acetalen zu Verfärbungen der erzeugten Produkte, die unerwünscht ist.

Es war deshalb die technische Aufgabe gestellt, ein Verfahren zur Verfügung zu stellen, das es ermöglicht, Acetale und/oder Aldehyde aus Carbonsäureestern auf einfache Weise zu entfernen.

Diese Aufgabe wird gelöst in einem Verfahren zur Reinigung von Aldehyde und/oder Acetale enthaltenden Carbonsäureestern, die durch Carbonylierung von olefinisch ungesättigten Verbindungen mit Kohlenmonoxid und Alkanolen erhalten worden sind, wobei man die Aldehyde und/oder Acetale enthaltenden Carbonsäureester mit Schwefelsäure, Phosphorsäure, Benzolsulfonsäure, Toluolsulfonsäure oder stark sauren Ionenaustauschern, gegebenenfalls unter Zusatz von Wasser, behandelt und nach Entfernen des abgespaltenen Alkanols die entstandenen Hochsieder bzw. Leichtsieder destillativ abtrennt.

Das neue Verfahren hat den Vorteil, daß die Ester auf einfache Weise von den verunreinigenden Aldehyden oder Acetalen befreit werden, die sonst nur unter erheblichem Aufwand mittels mehrstufiger Reinigungsverfahren abgetrennt werden können. Die nach dem erfindungsgemäßen Verfahren gereinigten Carbonsäureester zeichnen sich durch sehr hohe Reinheit aus, da die Entfernung der Acetale und der Aldehyde quantitativ erfolgt.

Der Erfindung liegt der allgemeine Erfindungsgedanke zugrunde, die nahe bei den Carbonsäureestern siedenden Acetale mit sauren Mitteln in Vinyläther zu überführen und diese als Leichtsieder abzutrennen oder die Acetale und Aldehyde beispielsweise die Aldehyde allein durch Aldolisierung in Hochsieder zu überführen und als solche abzutrennen. Demzufolge kann das Verfahren auch angewandt werden auf Kohlenwasserstoffe, Äther, Ketone, Halogenverbindungen oder aromatische Kohlenwasserstoffe, die mit Acetalen und/oder Aldehyden verunreinigt sind.

Bevorzugte Carbonsäureester erhält man durch Carbonylierung von $C_1$-$C_{12}$-Monoolefinen, $C_4$-$C_{12}$-Diolefinen, $C_5$-$C_{12}$-Cycloalkenen oder $C_3$-$C_{12}$-Alkenmonocarbonsäure-$C_1$-$C_8$-Alkylestern. Die Carbonylierung erfolgt nach bekannten Verfahren durch Umsetzen mit Kohlenmonoxid und $C_1$-$C_8$-Alkanolen insbesondere $C_1$ bis $C_4$-Alkanolen, z. B. bei Temperaturen von 100 bis 200 °C und unter Drücken von 50 bis 1 000 bar in Gegenwart von Carbonylkomplexen von Metallen der VIII. Gruppe des Periodensystems, insbesondere von Kobalt oder Rhodiumcarbonylkomplexen. Hieraus resultieren $C_3$-$C_{13}$-Monocarbonsäureester von Alkanolen mit 1 bis 8 Kohlenstoffatomen, $C_6$-$C_{14}$-Dicarbonsäureester von Alkanolen mit 1 bis 8 Kohlenstoffatomen oder Cycloalkancarbonsäureester mit 5 bis 12 Kohlenstoffatomen im Ring. Besonders bevorzugt sind gesättigte Mono- und Dicarbonsäureester mit den obengenannten Kohlenstoffzahlen. So hergestellte Ester enthalten als Nebenprodukte Aldehyde und/oder Acetale, wobei die Aldehyde die gleiche Kohlenstoffzahl aufweisen wie die entsprechenden Carbonsäuren. Die Acetale enthalten darüber hinaus noch die jeweiligen Reste, die den mitverwendeten Alkanolen entsprechen. Der Gehalt an Aldehyden und/oder Acetalen beträgt beispielsweise 0,1 bis 15 Gew.%. Geeignete Verfahren werden z. B. beschrieben in der US-PS 3 176 028 und DE-OS 16 18 156.

Besondere technische Bedeutung haben Adipinsäure-$C_1$-$C_4$-alkylester erlangt, die durch Carbonylierung von Butadien oder Pentensäure-$C_1$-$C_4$-alkylester mit Kohlenmonoxid und $C_1$-$C_4$-Alkanolen hergestellt worden sind. Ein typisches Gemisch enthält beispielsweise neben Adipinsäureester 9 bis 14 Gew.% Methylglutarsäureester, 2 bis 5 Gew.% Äthylbernsteinsäureester, 0,1 bis 0,3 Gew.% 5-Formylvaleriansäureester, sowie 0,2 bis 0,5 Gew.% 6,6-Dimethoxycapronsäureester. Geeignete Verfahren werden z. B. beschrieben in der US-PS 2 801 263 und DE-PS 27 13 195.

Geeignete stark saure Mittel sind vorzugsweise Schwefelsäure, Phosphorsäure, Benzolsulfonsäure, Toluolsulfonsäure oder stark saure Ionenaustauscher (vernetztes Polystyrol mit Sulfonsäuregruppen). Besonders bevorzugt werden stark saure Ionenaustauscher oder Schwefelsäure verwendet. Obzwar die stark sauren Mittel bereits in katalytisch wirksamen Mengen angewandt werden können, wendet man vorteilhaft bezogen auf den Gehalt an Aldehyden und/oder Acetalen 1 bis 50 Gew.% stark saure Mittel an. Die angewandte Menge ist jedoch nicht kritisch. Die Behandlung erfolgt vorteilhaft bei Temperaturen von 20 bis 200 °C. Besonders bewährt haben sich Temperaturen von 80 bis 140 °C.

Bei der Reinigung von nur Acetale enthaltenden Carbonsäureestern werden die Acetale vorteilhaft durch Behandeln mit den vorgenannten sauren Mitteln und bei den beschriebenen Temperaturen in Vinyläther und Alkanol gespalten. Da es sich um eine Gleichgewichtsreaktion handelt, ist es vorteilhaft, freigesetztes Alkanol beispielsweise durch Strippen mit Inertgas wie Stickstoff zu entfernen. Zweckmäßig wird die Behandlung in Abwesenheit von Wasser durchgeführt um die mögliche Verseifung des Vinyläthers zu Aldehyd und Alkanol zu vermeiden. Der Vinyläther wird anschließend durch Destillation abgetrennt.

Besondere technische Bedeutung haben Carbonsäureester, die nur Aldehyd oder sowohl Aldehyd als auch Acetal enthalten. Hierbei überführt man zweckmäßigerweise diese Verunreinigungen in Hochsieder. Zunächst werden die Acetale durch die vorgenannten sauren Mittel zu Vinyläther und Alkanol gespalten, wobei man zweckmäßigerweise wiederum die Alkanole z. B. durch Strippen mit Inertgas entfernt. Durch Mitverwendung von Wasser vorteilhaft 0,5 bis 5 Mol Wasser je Mol Acetal, insbesondere 0,8 bis 2 Mol Wasser je Mol Acetal werden die Vinyläther zu Aldehyd und Alkanol verseift. Es sei erwähnt, daß auch höhere Wassergehalte als die angegebenen, ohne Nachteil angewandt werden können, z. B. bis zu 10 Mol je Mol Acetal. Bei den angegebenen Temperaturen aldolysieren die Aldehyde und ergeben Hochsieder, die leicht abgetrennt werden können. Die Behandlung kann diskontinuierlich erfolgen. Vorteilhaft führt man die Behandlung kontinuierlich durch, wobei man in einer ersten Stufe bei den angegebenen Temperaturen, mit den vorgenannten stark sauren Mitteln und der angegebenen Wassermenge Acetale unter Entfernung des Alkanols spaltet und in einer zweiten Stufe die Aldolisierung zu Hochsiedern bei den angegebenen Temperaturen durchführt.

Die erfindungsgemäß gereinigten Ester eignen sich als Lösungsmittel. Adipinsäureester werden zu Adipinsäure verseift, das ein Ausgangsprodukt zur Herstellung von Polykondensaten wie Polyamid 6.6 ist.

Das Verfahren nach der Erfindung sei in folgenden Beispielen veranschaulicht :

Beispiel 1

In einem 2 l-Rundkolben mit Rückflußkühler und Gaseinleitung werden 1 000 g Adipinsäuredimethylester, der 0,75 % 6.6-Dimethoxycapronsäuremethylester enthält, mit 20 g wasserfreier Phosphorsäure bei 110° gerührt und das freigesetzte Methanol mit 50 l/h Stickstoff gestrippt. Nach 2,5 Stunden wird zuerst abgekühlt, dann von der Phosphorsäure abgetrennt und schließlich der organische Austrag bei 20 mbar fraktioniert destilliert. Es werden 983 g reiner Adipinsäuredimethylester erhalten, der nach gaschromatographischer Analyse keinen 6.6-Dimethoxycapronsäuremethylester mehr enthält.

Beispiel 2

In eine Kaskade bestehend aus einem beheizbaren 10 l-Rührkessel mit Kühler und Gaseinleitung, gefüllt mit 2,5 l eines makroporösen sauren Ionentauschers, und einem nachgeschalteten von oben durchströmten 1,8 m langen Strömungsrohr (Innendurchmesser 6 cm), gefüllt mit dem gleichen Ionentauscher, werden stündlich 2 l Adipinsäuredimethylester zugeführt. Der Adipinester ist mit 0,3 % 6.6-Dimethoxycapronsäuremethylester und 0,25 % 5-Formylvaleriansäuremethylester verunreinigt und hat einen Wassergehalt von 0,1 %. Rührkessel und Strömungsrohr werden auf 120° beheizt ; das im Rührkessel freigesetzte Methanol wird mit 100 l/h Rein-Stickstoff gestrippt. Das aus dem Strömungsrohr unten ausgetragene Gemisch aus Adipinsäuredimethylester und Hochsiedern wird einer Vakuumdestillation zugeführt. Der hieraus resultierende Adipinester ist vollständig von 6.6-Dimethoxycapronester und 5-Formylvalerianester befreit und hat eine Reinheit von 99,9 %.

## Ansprüche

1. Verfahren zur Reinigung von Aldehyde und/oder Acetale enthaltenden Carbonsäureestern, die durch Umsetzung von olefinisch ungesättigten Verbindungen mit Kohlenmonoxid und Alkanolen erhalten worden sind, dadurch gekennzeichnet, daß man die Aldehyde und/oder Acetale enthaltenden Carbonsäureester mit Schwefelsäure, Phosphorsäure, Benzolsulfonsäure, Toluolsulfonsäure oder stark sauren Ionenaustauschern, gegebenenfalls unter Zusatz von Wasser, behandelt und nach Entfernen des abgespaltenen Alkanols die entstandenen Hochsieder bzw. Leichtsieder destillativ abtrennt.

2. Verfahren nach Anspruch 1, dadurch gekennzeichnet, daß man 1 bis 50 Gew.% stark saure Mittel, bezogen auf den Gehalt an Aldehyden und/oder Acetalen anwendet.

3. Verfahren nach den Ansprüchen 1 und 2, dadurch gekennzeichnet, daß man die Behandlung bei 80 bis 140 °C durchführt.

4. Verfahren nach den Ansprüchen 1 bis 3, dadurch gekennzeichnet, daß man Acetale enthaltende Carbonsäureester in Abwesenheit von Wasser unter Entfernung des abgespaltenen Alkanols behandelt und den entstandenen Vinyläther durch Destillation abtrennt.

5. Verfahren nach den Ansprüchen 1 bis 3, dadurch gekennzeichnet, daß man Carbonsäureester, die Aldehyde oder Aldehyde und Acetale enthalten unter Mitverwendung von Wasser unter Entfernung des abgespaltenen Alkanols behandelt und die Carbonsäureester von den gebildeteten Hochsiedern durch Destillation abtrennt.

6. Verfahren nach den Ansprüchen 1 bis 5, dadurch gekennzeichnet, daß man Adipinsäuredimethylester der mit 5-Formylvaleriansäuremethylester und/oder 6.6-Dimethoxycapronsäuremethylester verunreinigt ist, als Ausgangsgemisch verwendet.

## Claims

1. A process for purifying carboxylic acid esters which have been obtained by reacting olefinically unsaturated compounds with carbon monoxide and alkanols and which contain aldehydes or acetals, wherein the said esters are treated with sulfuric acid, phosphoric acid, benzenesulfonic acid, toluenesulfonic acid or a strongly acidic ion-exchanger, with or without addition of water, and, after removal of the alkanol split off, the high-boilers or volatiles formed are separated off by distillation.

2. A process as claimed in claim 1, wherein from 1 to 50 % by weight, based on the content of aldehydes and/or acetals, of the strongly acidic agent is used.

3. A process as claimed in claims 1 and 2, wherein the treatment is carried out at from 80 to 140 °C.

4. A process as claimed in claims 1 to 3, wherein the carboxylic acid esters containing acetals are treated in the absence of water, with removal of the alkanol split off, and the resulting vinyl ether is separated off by distillation.

5. A process as claimed in claims 1 to 3, wherein carboxylic acid esters which contain aldehydes, or aldehydes and acetals, are treated in the presence of water, with removal of the alkanol split off, and the carboxylic acid esters are separated by distillation from the high-boilers formed.

6. A process as claimed in claims 1 to 5, wherein dimethyl adipate which is contaminated with methyl 5-formylvalerate and/or methyl 6,6-dimethoxycaproate is used as the starting mixture.

## Revendications

1. Procédé de purification d'esters d'acides carboxyliques contenant des aldéhydes et (ou) des acétals et préparés par réaction de composés à insaturation oléfinique avec des alcanols et l'oxyde de carbone, caractérisé en ce que l'on traite les esters d'acides carboxyliques contenant des aldéhydes et (ou) acétals avec de l'acide sulfurique, de l'acide phosphorique, de l'acide benzène-sulfonique ou de l'acide toluène-sulfonique ou avec des échangeurs d'ions fortement acides, éventuellement avec addition d'eau, on élimine les alcanols libérés, puis on sépare par distillation les produits à point d'ébullition élevé ou à bas point d'ébullition formés.

2. Procédé suivant la revendication 1, caractérisé en ce que les produits fortement acides sont mis en œuvre à raison de 1 à 50 % en poids par rapport à la teneur en aldéhydes et (ou) acétals.

3. Procédé suivant les revendications 1 et 2, caractérisé en ce que le traitement est réalisé entre 80 et 140 °C.

4. Procédé suivant les revendications 1 à 3, caractérisé en ce que les esters d'acides carboxyliques, contenant des acétals, sont traités en l'absence d'eau avec élimination concomitante de l'alcanol libéré, l'éther vinylique formé étant séparé par distillation.

5. Procédé suivant les revendications 1 à 3, caractérisé en ce que les esters d'acides carboxyliques, contenant des aldéhydes ou des aldéhydes et des acétals, sont traités en présence d'eau avec élimination concomitante de l'alcanol libéré, les esters d'acides carboxyliques étant ensuite séparés par distillation des produits à point d'ébullition élevé formés.

6. Procédé suivant les revendications 1 à 5, caractérisé en ce que l'on emploie comme mélange de départ de l'adipate de diméthyle, contenant comme impuretés de l'ester méthylique de l'acide formyl-5 valérique et (ou) de l'ester méthylique de l'acide diméthoxy-6,6 hexanoïque.